# FASCICULE DE BREVET EUROPEEN

(11) **EP 2 124 793 B1**
(45) Date de publication et mention de la délivrance du brevet: **22.08.2012**
(21) Numéro de dépôt: 08761812.0
(22) Date de dépôt: 29.01.2008
(51) Int. Cl.: A61B 18/20, A61B 17/04

(54) **ÉQUIPEMENT DE TRAITEMENT DE PLAIES**
VORRICHTUNG ZUR BEHANDLUNG VON WUNDEN
EQUIPMENT FOR TREATING WOUNDS

(30) Priorité: 29.01.2007 FR 0752934
(43) Date de publication de la demande: 02.12.2009
(73) Titulaire: EKKYO, 13857 Aix-en-Provence Cédex 3 (FR)
(72) Inventeur: CORNIL, Alain, F-13090 Aix-en-Provence (FR); CAPON, Alexandre, F-59237 Verlinghem (FR); GOSSE, Alban, F-13105 Mimet (FR); PERONNE, Patrick, F-13100 Aix-en-Provence (FR)
(74) Mandataire: Barbot, Willy
(86) Numéro de dépôt international: PCT/FR2008/000102
(87) Numéro de publication internationale: WO 2008/107563

(56) Documents cités:
- EP-A- 0 976 362
- WO-A-97/17025
- WO-A-2004/010885
- US-A- 5 156 613
- US-B1- 6 773 699

## Description

La présente invention concerne le domaine du traitement des plaies par un faisceau laser, et les équipements pour de tels traitements.

On connaît dans l'état de la technique le brevet FR9809557 concernant une méthode et un dispositif d'assistance à la fermeture de plaies associant un laser et un pansement adhésif transparent. Le pansement est destiné à rapprocher les berges d'une incision cutanée pendant que le laser est utilisé pour chauffer ce même tissu à travers le pansement et favoriser ainsi la cicatrisation.

Ce système d'une mise en oeuvre simple et rapide souffre cependant de plusieurs défauts :
- Lors de son utilisation en clinique humaine il s'est avéré que les incisions produisaient des exsudats se répandant entre le pansement et la peau éliminant ainsi l'adhésion du pansement dans la zone autour de l'incision. Or pour obtenir des résultats satisfaisants il est impératif que les berges de la plaie soient maintenues en contact étroit pendant tout le temps ou la plaie n'est pas totalement refermée. A défaut, il en résulte une fibrose plus importante dans l'espace ouvert entraînant l'apparition de cicatrice et un risque accru d'infection.
- Compte tenu de la longueur et des formes courbes de certaines plaies chirurgicales, il est très difficile d'appliquer un pansement unique de façon homogène sur ces plaies. Il faut alors pour garantir un bon affrontement des plaies, utiliser de multiples bandelettes de pansement placées l'une à côté de l'autre, augmentant ainsi le risque infectieux postopératoire.

Il est donc avantageux de proposer une méthode de fermeture de la plaie qui ne constitue pas un obstacle à l'écoulement des exsudats tout en réalisant une bonne approximation des berges. L'utilisation de suture ou d'agrafes semblent pouvoir répondre à ce problème. Cependant le matériau utilisé devra avoir un coefficient d'absorption de l'énergie de la source inférieur ou égal à celui de la peau dans laquelle il est implanté afin d'éviter un échauffement plus important de celui-ci.

Une autre limitation du brevet concerne la sécurisation du laser utilisé. Deux autres brevets en cours de dépôt (demandes FR0600160 et FR0651942) décrivent des dispositifs et méthodes permettant de sécuriser le déclenchement du laser en le conditionnant à la détection du pansement par ce même laser.

De manière plus générale, les lasers utilisés pour des applications dermatologiques ne disposent d'aucun système de détection automatique de la zone à traiter. Notre dispositif ainsi décrit permet d'assurer cette fonction et de sécuriser ainsi le fonctionnement de ce type de laser tout en n'obstruant pas la zone traitée.

Le dispositif proposé selon l'invention combine une source d'énergie pour l'activation d'un effet biochimique dans la peau. La source d'énergie est associée à un dispositif d'identification avec ou sans contact placé à proximité de la zone à traiter mais ne la recouvrant pas. La source d'énergie ne peut être activée que lorsque le capteur qui lui est associé détecte le dispositif d'identification.

Pour être utilisable dans le cas de fermeture de plaies, le dispositif est associé à un élément de maintien mécanique implanté dans l'épaisseur de la peau permettant d'apposer les berges de la plaie traitée, dont le matériau et les dimensions sont choisies de manière à ne pas engendrer d'échauffement excessif ni de brûlure du tissu traité sous l'effet de la source d'énergie.

Grâce à l'invention, nous maintenons un bon affrontement des plaies pendant toute la durée de la cicatrisation tout en permettant la sécurisation et le paramétrage du laser en fonction du type d'incision et de tissu traité. La demande de brevet WO97/17025 décrit un système pour le traitement des plaies de la peau comprenant une source d'énergie adaptée pour l'activation biochimique de la cicatrisation et au moins un moyen d'intéraction entre la source d'énergie et le patient. Cependant, elle ne décrit pas de système comprenant un moyen d'identification intéragissant sans contact avec un capteur commandant le fonctionnement de la source d'énergie seulement lorsque la distance entre le capteur et ledit moyen d'identification est inférieure à une valeur seuil.

Plus précisément, l'invention a pour objet un système Plus précisément, l'invention a pour objet un système pour le traitement dermatologique comprenant une source d'énergie adaptée pour l'activation biochimique de la cicatrisation et au moins un moyen d'interaction entre la source d'énergie et le patient, caractérisé en ce que ledit moyen d'interaction est constitué par un support adhésif continu apte à être placé à proximité de la zone à traiter, ledit support étant muni d'un moyen d'identification interagissant sans contact avec un capteur commandant le fonctionnement de la source d'énergie seulement lorsque la distance entre le capteur et ledit moyen d'identification est inférieure à une valeur seuil.

Selon des modes de réalisation particuliers :
- Le support adhésif comprend au moins 2 moyens d'identification espacés d'une longueur au moins égale à un multiple entier de la largeur de la zone traitée par un tir individuel.
- Les moyens d'identification sont espacés d'une longueur égale à la largeur de la zone traitée par un tir individuel;
- Le moyen d'identification interagit avec la source d'énergie grâce à un contact;
- Les paramètres de fonctionnement de la source d'énergie sont mémorisés dans le moyen d'identification et communiqués au dispositif de commande de la source d'énergie;
- Une information caractérise l'état de la zone à traiter, à proximité de laquelle un support adhésif muni d'un moyen d'identification est placé, l'information étant enregistrée dans le moyen d'identification, et étant relative à l'un des états suivants :
   - la zone à traiter n'a pas été traitée,
   - la zone à traiter est en cours de traitement,
   - le traitement de la zone à traiter est terminé;
- Le dispositif de commande recevant l'information relative à l'état de la zone à traiter comporte des moyens d'empêcher une seconde utilisation du support adhésif muni
d'un moyen d'identification lorsque celui-ci a déjà été utilisé une première fois pour traiter une zone à traiter;
- Le système pour le traitement dermatologique est associé à un dispositif de fermeture de plaie placé dans les berges de la plaie dont le coefficient d'absorption est inférieur à celui de la peau pour la source d'énergie considérée;
- Le système pour le traitement dermatologique comporte une pièce à main incorporant une source de rayonnement électromagnétique pour l'activation biochimique de la cicatrisation et un moyen d'interaction avec ledit support adhésif;
- Le système pour le traitement dermatologique comporte une pièce à main incorporant une source laser et un moyen d'interaction avec ledit support adhésif;
- Le système pour le traitement dermatologique comporte une source d'énergie commandée par un calculateur recevant un signal provenant d'un capteur apte à interagir avec le moyen d'identification du pansement ou du support adhésif;
- La pièce à main comprend un lecteur radiofréquence;
- La portée de l'antenne du lecteur radio-fréquence est inférieure à 5 mm.

L'invention concerne aussi un support adhésif d'un système pour le traitement dermatologique défini ci-dessus, comprenant des moyens empêchant une seconde utilisation du support adhésif si celui-ci a déjà été utilisé pour le traitement d'une zone à traiter.

Selon des modes de réalisation particuliers :
- Le support adhésif comprend un transpondeur;
- Le transpondeur du support adhésif fonctionne dans une bande de fréquence comprise entre 13 et 14 Mhz;
- Le support adhésif comprend sur sa surface supérieure des marquages espacés d'une longueur égale à la largeur de la zone traitée par un tir individuel;
- Le support adhésif est opaque.

Il est également décrit une méthode de traitement dermatologique adaptée pour l'activation biochimique de la cicatrisation chez un patient comprenant les étapes suivantes :
(i) positionner, à proximité de la zone à traiter, un support adhésif muni d'un moyen d'identification interagissant sans contact avec un capteur commandant le fonctionnement d'une source d'énergie, et
(ii) positionner ladite source d'énergie, comprenant un capteur commandant son fonctionnement, à une distance entre ledit capteur et ledit moyen d'identification inférieur à une valeur seuil de sorte d'activer ladite source d'énergie et de permettre l'activation biochimique de la cicatrisation au niveau de la zone traitée.

La méthode de traitement dermatologique est adaptée pour la fermeture de plaies.

La méthode de traitement dermatologique comprend une étape préalable de mise en place d'un dispositif de fermeture de plaie, placé dans les berges de la plaie dont le coefficient d'absorption est inférieur à celui de la peau pour la source d'énergie considérée.

Dans un mode de réalisation préféré, le dispositif de fermeture de plaies se compose d'un support adhésif muni d'un tag RFID composé d'une mémoire électronique associée à une antenne, d'une pièce à main laser muni d'un système de détection RFID et d'un fil de suture continu positionné dans l'épaisseur de la plaie et sur toute sa longueur.

Le tag RFID, idéalement compatible avec la norme ISO 15693, est composé d'une puce électronique classique, par exemple de type NXP i-code SLI ou SLI-S, Inside Contactless Picopass 2k ou ST LRI 2K. Les tags ainsi que le lecteur RFID de la pièce à main utilisent une bande de fréquence entre 10 et 20 MHz, de préférence une bande de fréquence entre 13 et 14 MHz, permettant ainsi des lectures à faibles distances. Avantageusement, l'antenne des tags et celle des lecteurs RFID sont spécialement conçues pour sécuriser le dispositif et permettre ainsi de stopper le tir, dès que le tag sort du champ du lecteur (distance supérieure à 10mm).

Avantageusement, ledit support adhésif peut être décliné en plusieurs longueurs (4, 10 et 20 cm par exemple) et peut également prendre la forme d'un rouleau débité de sorte d'obtenir un support adhésif présentant la longueur souhaitée.

L'invention sera mieux comprise à la lecture de la description qui suit, se référant aux figures annexées où :
- la figure 1 représente un schéma de principe du système selon une variante de réalisation,
- la figure 2 représente une vue schématique du support adhésif,
- la figure 3 représente une vue en perspective du support adhésif communiquant avec la pièce à main,
- la figure 4 représente une vue du support adhésif,
- la figure 5 représente un schéma de communication entre le tag RFID et la pièce à main,
- les figures 6a, 6b et 6c décrivent des courbes de Températures en fonction du temps de chauffe du système,
- la figure 7 représente un organigramme de fonctionnement du système, et
- la figure 8 représente un organigramme de la gestion de l'autorisation de tir.

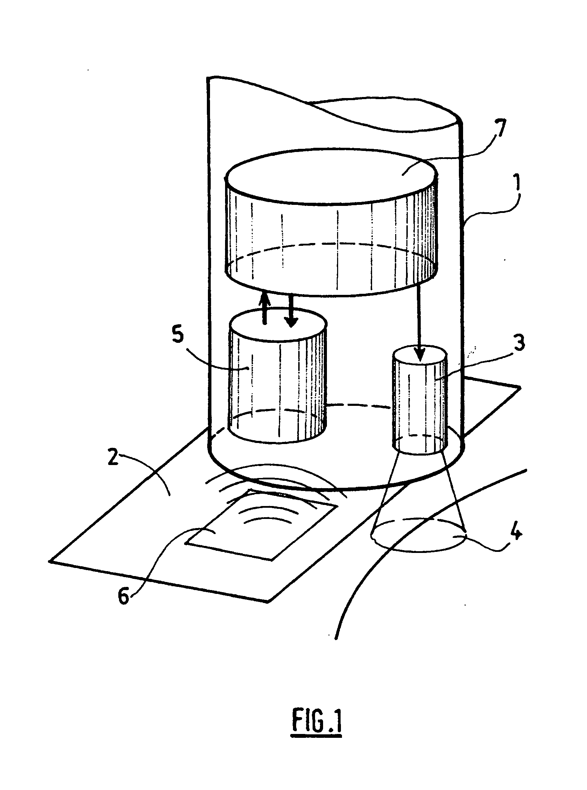

En référence à la figure 1, le dispositif est constitué de deux parties principales :
- une pièce à main (1),
- un support adhésif (2)

La pièce à main (1) comprend une source d'excitation apte à provoquer un effet biochimique sur la plaie (3). Il s'agit dans l'exemple décrit d'une source laser (3) produisant un spot (4) lorsqu'il est projeté sur la surface de la peau du patient.

La pièce à main (1) comprend également un lecteur (5) interagissant avec un marqueur (6) solidaire du support (2).

La pièce à main (1) comprend en plus un circuit électronique (7) de commande. Ce circuit commande conditionnellement le fonctionnement du laser (3), notamment son activation lorsque la pièce à main est située à proximité de la peau du patient et de la zone à traiter, ainsi que la puissance du laser.

La détection de la proximité de la zone à traiter est réalisée par analyse des signaux provenant du lecteur (5) en fonction des échanges avec le marqueur (6).

Les paramètres de tir du laser sont commandés en fonction de la nature des informations transmises par le marqueur (6), qui contient des paramètres permettant d'adapter la puissance, la durée et le nombre des tirs en fonction des choix de l'utilisateur. L'utilisateur sélectionne le support en fonction de la nature du traitement à effectuer, et le support sélectionné délivre au récepteur (5) un signal ou une information qui pilotera les tirs du laser (3). Les supports adhésifs sont disponibles en plusieurs variantes dépendant par exemple:
- de l'indication d'utilisation,
- du type de peau du patient (phototype, épaisseur, âge, localisation)
Ils permettent à l'utilisateur de répondre directement à son besoin sans avoir à se soucier des paramètres à utiliser pour le traitement.

Le support (2) est constitué par exemple par un timbre (ou, en anglais « patch ») formé par un morceau de tissu adhésif comprenant une étiquette radiofréquence (en anglais « RFID ») constituée par un dessin d'antenne permettant un couplage inductif pour l'alimentation d'un composant HF, et la transmission du signal haute fréquence émis par ce composant.

Le support est positionné à proximité de la zone à traiter de manière à ce que la pièce à main soit à portée d'interaction avec l'étiquette pendant la durée du traitement.

La distance entre le support et la pièce à main est comprise entre un et quinze centimètres. L'évaluation de la distance est réalisée par une portée limitée des moyens d'interaction entre le support et la pièce à main, ou par un télémètre, par exemple un télémètre à ultrasons, intégré dans la pièce à main.

Le système comprend en outre un fil de suture. Ce fil de suture est placé de manière à affronter les berges parfaitement. Le matériau de ce fil est choisi de façon à ce que son coefficient d'absorption optique à la longueur d'onde du laser ne soit pas supérieur à celui du tissu cutané, par exemple un copolymère de type PGLA (acide polyglycolique / polylactique).

Le support adhésif est placé à proximité immédiate de la plaie de telle façon que le détecteur de la pièce à main laser est à portée de détection du support et que le faisceau du laser est perpendiculaire à la zone à traiter.
Cette proximité permet de sécuriser le dispositif en stoppant le tir immédiatement si le tag compris dans le support adhésif sort du champ du lecteur compris dans la pièce à main. Elle est donc portée au maximum à 10 mm. Au-delà, le support adhésif ne peut plus jouer son rôle de verrouillage et de sécurisation du dispositif. La distance du support adhésif par rapport à l'incision à traiter est également importante. Il doit être placé longitudinalement à proximité, idéalement moins de 10mm, de l'incision de façon à ne pas perturber le traitement de l'incision par le dispositif d'une part, ne pas causer d'étirements involontaires de l'incision qui pourraient nuire au rapprochement des berges d'autre part.

Dans le cas de lésions longues à traiter, l'adhésif est muni de plusieurs tags espacés d'une longueur égale à un multiple entier de la largeur du faisceau de traitement, de façon à contrôler l'espacement des tirs, le temps de tir effectif ainsi que l'énergie total délivrée.

Le support adhésif a un triple rôle :
- la sécurisation du dispositif : Le fonctionnement du dispositif est immédiatement bloqué dès que le tag inclus dans le support adhésif sort du champ du lecteur RFID compris dans la pièce à main
- la définition des paramètres de traitement : L'utilisateur choisit le support adhésif en fonction de l'indication et du type de peau du patient, et les tags pré-programmés dans le support adhésif envoient directement les paramètres adéquats au dispositif.
- la protection de son intégrité (usage unique) : Lors de son utilisation, le dispositif inscrit les informations relatives à l'état du traitement sur les tags contenus dans le support adhésif. Les états de traitement sont :
- la zone à traiter n'a pas été traitée,
- la zone à traiter est en cours de traitement, et le traitement de la zone à traiter est terminé.

Dès lors que le traitement est terminé, le dispositif de commande recevant l'information relative à l'état de la zone à traiter comporte des moyens d'empêcher une seconde utilisation du support adhésif muni d'un moyen d'identification lorsque celui-ci a déjà été utilisé une première fois pour traiter une zone à traiter. Ce principe de verrouillage des tags permet donc de s'assurer que chaque support adhésif ne sera utilisé qu'une seule fois. L'avantage direct de ce système est l'impossibilité :
- d'appliquer un traitement deux fois au même endroit (élimination du risque de surdosage),
- de ré-utiliser un support adhésif livré stérile qui ait déjà été utilisé

Il pourra être prévu que les moyens d'empêcher une seconde utilisation du support adhésif soient directement compris dans le moyen d'identification lui-même.

Toujours dans le but d'aider l'utilisateur dans le bon déroulement de l'acte chirurgical ou médical, les supports adhésifs ont un quatrième rôle d'aide et de suivi de l'acte.

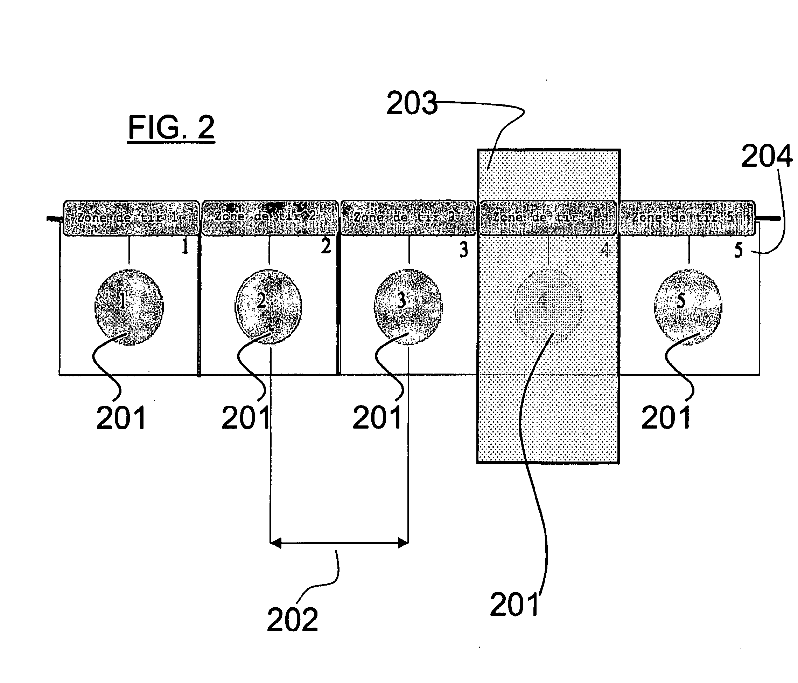

En effet, les supports adhésifs disposent d'un système de guidage permettant à l'utilisateur de suivre le traitement tout le long d'une incision par exemple (fig. 2). Ce système de guidage est mis en place grâce à une numérotation spécifique des tags, une règle ou graduation. Ce système visuel permet d'une part à l'utilisateur de suivre le traitement, et d'autre part d'avoir un moyen mnémotechnique pour connaître l'endroit où il s'est arrêté en cas d'interruption involontaire du traitement.

En référence à la figure 2, dans le cas du traitement d'une zone nécessitant plusieurs tirs contigus, le support adhésif contient plusieurs tags (201) espacés d'une distance équivalente à la largeur du faisceau de tir (202). De cette façon, le tag permet de contrôler non seulement l'activation du laser mais également le positionnement du laser (203) en cas de tirs contigus et le temps de tir effectif et donc la dose d'énergie délivrée pour chaque tir.

Le support adhésif est muni de marqueurs visuels (204) permettant le placement précis du laser au-dessus du tag.

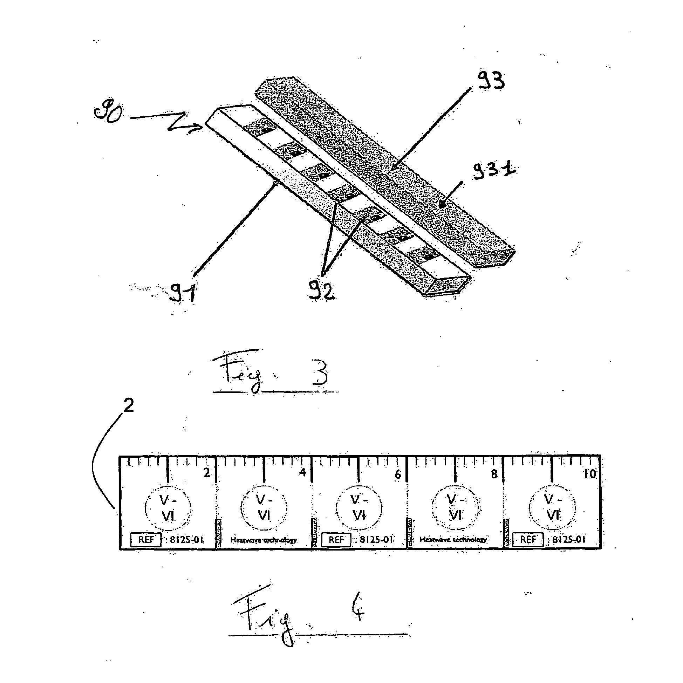

En référence à la figure 3, il est prévu de sécuriser et paramétrer le traitement grâce à un support adhésif (90) contenant une puce RFID qui communiquera à la pièce à main les différents paramètres de fonctionnement en fonction de l'indication d'utilisation et du phototype du patient à traiter. Ce support adhésif (90), qui peut être décliné en plusieurs longueurs (4, 10 et 20 cm par exemple), est collé à environ 5 mm de la zone à traiter.

Le support adhésif (90) est composé de deux sous-supports adhésifs : un sous-support adhésif (91) en double face et l'autre sous-support adhésif (93) en simple face. Ces deux sous-supports prennent en sandwich des tags RFID (92) qui seront positionnées tous les 2 cm. Le support adhésif (90) peut avoir par exemple une largeur de 2 cm.

Le sous-support adhésif inférieur (91) est en contact avec la peau du patient. En conséquence, il doit répondre aux problématiques de biocompatibilité et de maintien suffisant sur la peau durant son utilisation. Le sous-support adhésif inférieur (91) doit pouvoir coller sur la peau du patient et être assemblé aussi bien avec le tag RFID qu'avec le sous-support adhésif supérieur (93). De ce fait, le sous-support adhésif inférieur est à double face adhésive.

La fabrication des supports adhésifs (90) comporte une étape de stérilisation.

Le sous-support adhésif supérieur (93) doit être une adhésive simple face afin de permettre l'assemblage avec les tags RFID (92) et le sous-support adhésif inférieur (91). En référence à la figure 4 et sur sa partie supérieure (93), il est prévu d'imprimer des informations comme le phototype, le nom de l'entreprise, une graduation qui permettra au praticien de savoir où il en est par rapport au traitement ainsi que la référence du produit.

Le sous-support adhésif supérieur (93) est tel qu'il n'altère pas la communication RFID entre le lecteur RFID de la pièce à main et les tags RFID (92).

Les tags RFID (92) sont conformes à la norme ISO 15693. Il est d'ailleurs prévu d'implémenter les parties obligatoires et optionnelles telles que définies par cette présente norme. La distance de lecture du tag RFID étant une donnée critique, les tags RFID (92) selon l'invention sont tels qu'ils permettent de fixer cette valeur une fois le couple «tag RFID - Lecteur RFID» choisi. Les tags RFID peuvent avoir par exemple une taille de 14 mm x 14 mm pour une épaisseur inférieure à 1 mm. Il est prévu que la capacité de stockage de l'EEPROM du tag RFID soit au minimum de 1024 octets. De plus, un processus de stérilisation à l'oxyde d'éthylène est prévu.

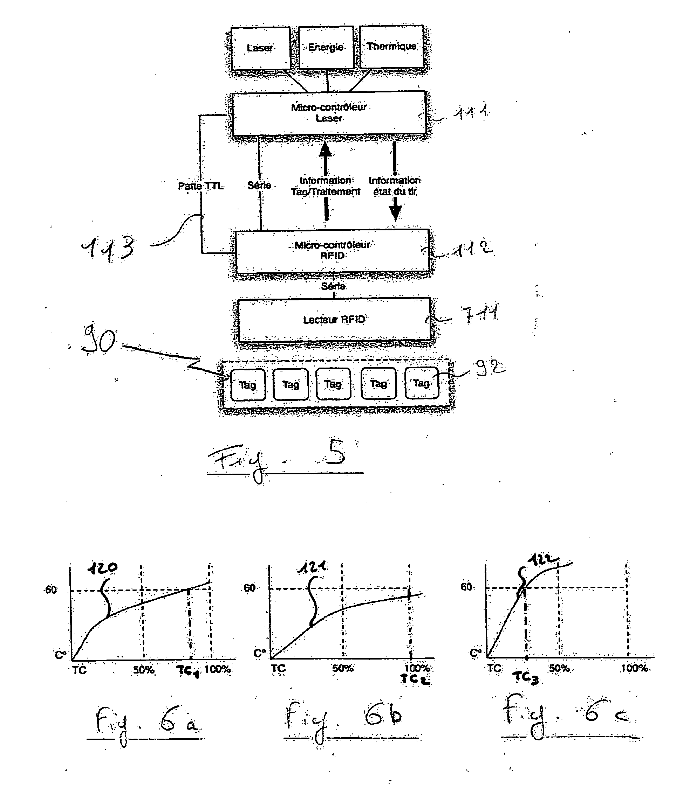

La figure 5 décrit un procédé de communication entre le tag RFID et la pièce à main comme suit : La pièce à main comprend deux microcontrôleurs (111) et (112). Le microcontrôleur (111) est responsable de la gestion du laser, de l'énergie, du contrôle thermique et de l'IHM ; le microcontrôleur (112) de la gestion des supports adhésifs 90. La responsabilité de la gestion des tirs est, quant à elle, partagée par les deux microcontrôleurs (111) et (112). Le tir peut être arrêté par le microcontrôleur (111), à cause d'une température trop élevée ou bien après une demande d'arrêt d'urgence. La reprise du traitement est contrainte par ces conditions d'arrêt.

En référence aux figures 6a, 6b et 6c, un pyromètre de la pièce à main, géré par le microcontroleur (111), arrête le tir si la température de la peau est supérieure à une température critique, 60°C par exemple. Ces figures sont des graphes présentant des courbes (120), (121) et (122) de température (degré Celsius en ordonnées) en fonction du temps de chauffe TC en abscisse ; TC1, TC2 et TC3 représentent les temps auxquels est arrêté le tir. Si le tir a été arrêté par le pyromètre, le tir peut à nouveau être autorisé une seule fois au bout d'un temps de sécurité (par exemple 5 secondes) si durant le premier tir, le temps de tir est inférieur ou égal à la moitié de la durée du tir donnée en paramètre (géré par le microcontrôleur (112)). C'est le cas de la figure 6c. Ainsi les figures présentent les cas suivants :
- la figure 6a présente le cas où le tir est arrêté (TC1) au-delà de 50% de la durée du tir en paramètre ; dans ce cas le tir ne peut être repris,
- la figure 6b présente le cas où le tir n'est pas arrêté jusqu'au bout de la durée du tir en paramètre (TC2), et
- la figure 6c présente le cas où le tir est arrêté (TC3) avant 50% de la durée de tir en paramètre ; dans ce cas le tir peut être repris.

Si le pyromètre se déclenche au-delà de 50% (fig. 6a) de la durée normale du tir, un bip doit signaler une fin de tir normal, ceci étant géré par le microcontrôleur (111). Si le pyromètre se déclenche en deçà de 50%, un élément de l'Interface Homme-Machine (IHM) montre à l'utilisateur qu'il peut récupérer le tir.

Si l'utilisateur décroche (arrêt, volontaire ou non, dû à l'utilisateur) et que le temps d'arrêt est inférieur ou égal à 75% de la durée du tir en paramètre, une reprise du tir peut être autorisée une seule fois au bout de 5 secondes et ce, durant 60 minutes.

En cas de déclenchement de l'arrêt d'urgence, il peut être prévu une procédure de vérification devant être suivie par l'utilisateur.

La présence d'un tag RFID (92), détecté par le lecteur RFID de la pièce à main, est indiquée par la patte TTL (113) du microcontrôleur (112). La présence d'un tag RFID (92) ne donne pas d'information concernant son état. Pour connaître l'état du tag RFID (92), il faut envoyer un message de lecture des paramètres au microcontrôleur (112). Cette commande permet de connaître l'état du traitement du tag RFID (92) présent dans le champ du lecteur RFID (711). Les différents états d'un tag RFID (92) sont :
- Traité
- OK (Non Traité)
- Reprise immédiate (identique à non traité)
- Attente avant reprise (5 secondes)

Le microcontrôleur (111) reconnaît cinq messages en provenance du microcontrôleur (112) :
- INIT : Initialisation de l'horloge et de l'identifiant de la pièce à main,
- PARAM : Récupérer l'état du tag RFID 92, la puissance et la durée du tir, le dispositif ne pouvant tirer que si l'état de PARAM est OK.
- START Début de tir,
- STOP Fin du tir, et
- PAUSE Arrêt prématuré du tir,

Au démarrage de la pièce à main, le microcontrôleur (111) initialise l'horloge du microcontrôleur (112) avec le message INIT. Les deux horloges se trouvent synchronisées. Au front montant de la patte TTL (113) du microcontrôleur (112), le microcontrôleur (111) demande les paramètres du tir avec le message PARAM. Selon la réponse du microcontrôleur (112), l'IHM est mise à jour et le tir est autorisé. Pendant le tir, le microcontrôleur (111) indique l'avancement du tir au microcontrôleur (112) avec les messages START, STOP et PAUSE. Le microcontrôleur (112) met à jour les informations des tags RFID (92) en fonction de ces messages. L'horloge de la pièce à main est gérée par l'un des microcontrôleurs (111) ou (112), indifféremment choisi par l'homme du métier. L'Horloge ne contient pas la date et l'heure exacte, mais une information de datation relative au démarrage de la pièce à main. Elle a une fonction de chronomètre et peut être réinitialisée suite à une interruption d'alimentation prolongée de la batterie. Dans ce cas, si un tag RFID (92) a été traité, sa date de début de traitement est postérieure à la date courante. Même si le traitement n'est pas terminé, le tag RFID (92) est considéré comme traité.

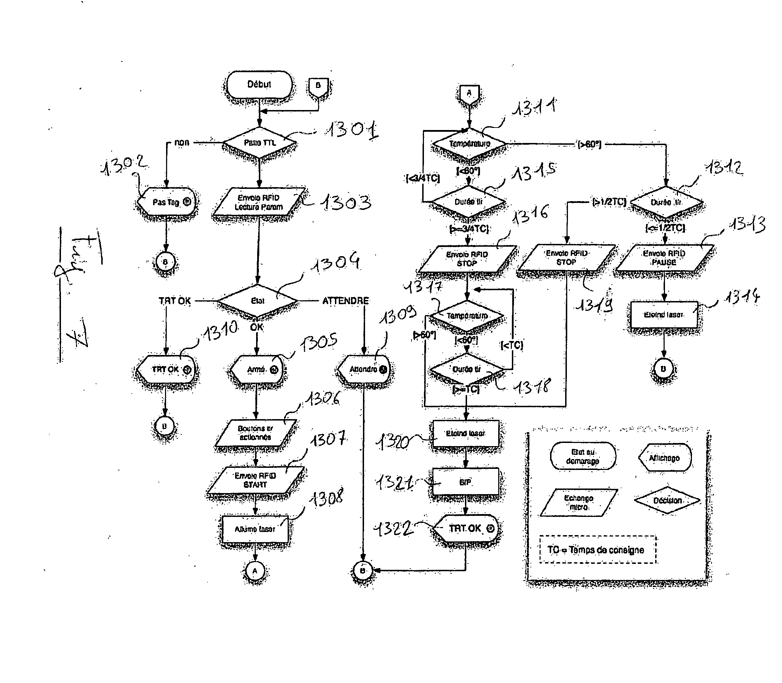

La figure 7 représente un algorithme de gestion de l'autorisation de tir du microcontrôleur (111) et un algorithme de gestion de l'autorisation de tir du microcontrôleur (112). Tous deux ont été détaillés précédemment. Initialement soit un tag RFID est détecté (1301) soit il n'est pas détecté (1302). Si un tag RFID est détecté (1301), une lecture des paramètres est initiée (1303) pour un diagnostique de l'état du tag RFID (1304). Si l'état est OK alors la pièce à main indique via l'IHM qu'il « armé » (1305) et, si le bouton de tir est actionné (1306), alors le microcontrôleur (111) envoie au microcontrôleur (112) le message START (1307) indiquant le début d'un tir. Enfin le laser est allumé (1308). Si le message est ATTENDRE, alors la pièce à main indique qu'il est « en attente » (1309) et revient au départ. Si le message est TRT OK (traité) alors la pièce à main indique que la zone a été traitée (1310) et revient au départ.

Si un tir est initié (bloc A), une mesure (1311) de la température est effectuée. Si cette température est supérieure à 60°, alors une mesure du temps (1312) est également effectuée. Si ce temps est inférieur à 50% de la durée du tir en paramètre, alors un message PAUSE (1313) est envoyé au microcontrôleur (112), et le laser est éteint (1314).

Si la température mesurée (1311) est inférieure à 60°, alors une mesure du temps (1315) est également effectuée. Si ce temps est inférieur à 75% de la durée du tir en paramètre, alors on recommence une mesure de la température (1311). Si, par contre, le temps est supérieur à 75% de la durée du tir en paramètre TC, un message STOP (1316) est envoyé au microcontrôleur (112). Une mesure de la température est alors effectuée (1317). Si la température est inférieure à 60°, alors on effectue une vérification sur la durée de tir (1318) ; si la durée du tir est inférieure à la durée du tir en paramètre TC (signifiant que le tir n'est pas encore terminé), alors on re-effectue une vérification sur la température (1317). Cette boucle (1317 et 1318) continue jusqu'à ce que la température dépasse 60° ou jusqu'à ce que la durée de tir atteigne ou dépasse TC. Dans les deux cas, on éteint le laser (1320), on envoie un bip (1321), ou tout autre moyen d'indication utilisant l'IHM, afin de prévenir l'utilisateur de la fin du traitement de cette zone et on affiche un message de fin de traitement (1322). Une nouvelle mesure du temps est effectuée (1312) ; si la durée de tir est supérieure à 50% de TC, alors un message STOP est envoyé au microcontrôleur (1319), puis on éteint le laser (1320). Si la durée du tir est inférieure ou égale à 50% de TC alors un message PAUSE est envoyé au microcontrôleur (1314) puis le laser est éteint.

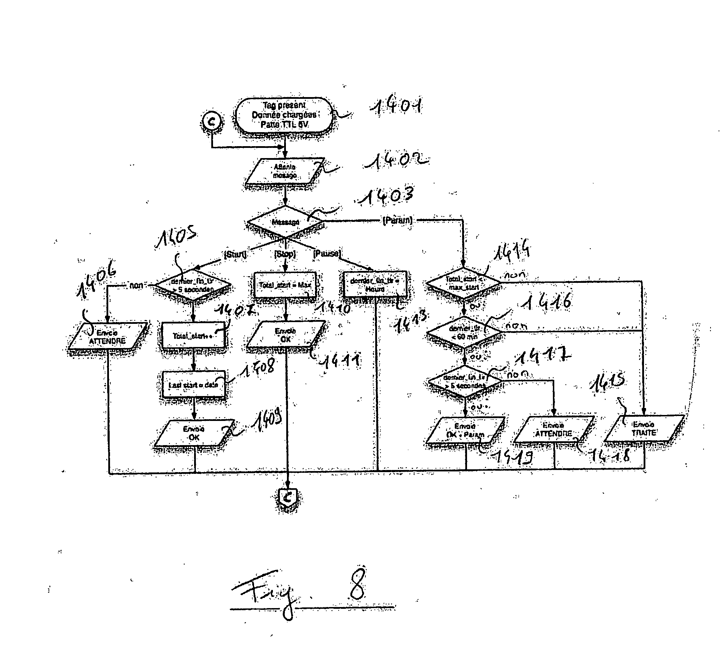

En référence à la figure 8, nous allons décrire un algorithme de gestion de l'autorisation de tir du microcontrôleur (111). si un tag RFID est détecté (1401), le microcontrôleur (111) se met en attente d'un message (1402) de la part du microcontrôleur (112). Lorsqu'un message (1403) est reçu, il est analysé. Si le message est START, et que le dernier tir date d'il y a moins de 5 secondes (1405), alors le microcontrôleur (111) envoie le message ATTENDRE (1406). Si, par contre, le dernier tir date d'il y a plus de 5 secondes, une variable Total_Start (indiquant le nombre de tirs effectués) est incrémentée d'une unité (1407) et une variable Last_Start (indiquant la date du dernier tir) est mise à jour (1408). Enfin un message OK est envoyé (1409).
Si le message reçu est STOP, alors la variable Total_Start est fixée à un maximum prédéterminé (1410) et le message OK est envoyé (1411).

Si le message reçu est PAUSE, on enregistre la date de fin de tir (1413).

Enfin, si le message reçu est PARAM, on analyse la variable Total_Start (1414). Si cette dernière est inférieure au maximum prédéfini, alors le message TRAITE est envoyé (1415). Dans le cas contraire, on analyse la variable Last_Start (1416), si ce dernier est supérieur à 60 minutes, alors le message TRAITE est envoyé (1415). Dans le cas contraire, on vérifie que le dernier tir date d'il y a plus de 5 secondes (1417); si ce n'est pas le cas, on envoie le message ATTENDRE (1418). Si, par contre, le dernier tir date d'il y a plus de 5 secondes, le message OK est envoyé et une lecture des paramètres est effectuée (1419). Les informations contenues dans le tag RFID peuvent être choisies parmi les suivantes : identifiant unique du tag RFID, date de fabrication, date de péremption, identifiant de paramétrage de la pièce à main en fonction du type de peau, numéro de lot de fabrication du support adhésif (90).

L'ensemble des chiffres (pourcentages, température, ...) de la description ne sont données qu'à titre purement indicatif afin d'orienter la réalisation de l'invention. L'homme du métier pouvant utiliser d'autres chiffrages déterminés, par exemple, par expérimentation sans sortir du cadre de l'invention.

## Revendications

1. - Système pour le traitement dermatologique comprenant une source d'énergie (3) adaptée pour l'activation biochimique de la cicatrisation et au moins un moyen d'interaction (2) entre la source d'énergie et le patient,
ledit moyen d'interaction (2) étant constitué par un support adhésif continu apte à être placé à proximité de la zone à traiter, **caractérisé en ce que** ledit support est muni d'un moyen d'identification (6) interagissant sans contact avec un capteur (5) commandant le fonctionnement de la source d'énergie seulement lorsque la distance entre le capteur et ledit moyen d'identification est inférieure à une valeur seuil.

2. - Système pour le traitement dermatologique selon la revendication 1, **caractérisé en ce que** le support adhésif (2) comprend au moins 2 moyens d'identification espacés d'une longueur au moins égale à un multiple entier de la largeur de la zone traitée par un tir individuel.

3. - Système pour le traitement dermatologique selon la revendication 2, **caractérisé en ce que** les moyens d'identification sont espacés d'une longueur égale à la largeur de la zone traitée par un tir individuel.

4. - Système pour le traitement dermatologique selon l'une quelconque des revendications précédentes, **caractérisé en ce que** le moyen d'identification interagit avec la source d'énergie grâce à un contact.

5. - Système pour le traitement dermatologique selon l'une quelconque des revendications précédentes, **caractérisé en ce que** les paramètres de fonctionnement de la source d'énergie sont mémorisés dans le moyen d'identification et communiqués au dispositif de commande de la source d'énergie

6. - Système pour le traitement dermatologique selon l'une quelconque des revendications précédentes, **caractérisé en ce qu'**une information caractérise l'état de la zone à traiter, à proximité de laquelle un support adhésif (2) muni d'un moyen d'identification est placé, l'information étant enregistrée dans le moyen d'identification, et étant relative à l'un des états suivants :
- la zone à traiter n'a pas été traitée,
- La zone à traiter est en cours de traitement
- le traitement de la zone à traiter est terminé.

7. - Système pour le traitement dermatologique selon la revendication 6, **caractérisé en ce que** le dispositif de commande recevant l'information relative à l'état de la zone à traiter comporte des moyens d'empêcher une seconde utilisation du support adhésif muni d'un moyen d'identification lorsque celui-ci a déjà été utilisé une première fois pour traiter une zone à traiter.

8. - Système pour le traitement dermatologique selon l'une quelconque des revendications précédentes, **caractérisé en ce qu'**il est associé à un dispositif de fermeture de plaie placé dans les berges de la plaie dont le coefficient d'absorption est inférieur à celui de la peau pour la source d'énergie considérée.

9. - Système pour le traitement dermatologique selon l'une quelconque des revendications précédentes, **caractérisé en ce qu'**il comporte une pièce à main (1) incorporant une source de rayonnement électromagnétique pour l'activation biochimique de la cicatrisation et un moyen d'interaction avec ledit support adhésif (2).

10. - Système pour le traitement dermatologique selon l'une quelconque des revendications précédentes, **caractérisé en ce qu'**il comporte une pièce à main (1) incorporant une source laser (9) et un moyen d'interaction avec ledit support adhésif (2)

11. - Système pour le traitement dermatologique selon l'une quelconque des revendications précédentes, **caractérisé en ce qu'**il comporte une source d'énergie commandée par un calculateur recevant un signal provenant d'un capteur apte à interagir avec le moyen d'identification du pansement ou du support adhésif (2)

12. - Système pour le traitement dermatologique selon la revendication 9, **caractérisé en ce que** la pièce à main (1) comprend un lecteur radio-fréquence.

13. - Système pour le traitement dermatologique selon la revendication 12, **caractérisé en ce que** la portée de l'antenne du lecteur radio-fréquence est inférieure à 5 mm.

14. - Support adhésif (2) d'un système pour le traitement dermatologique selon l'une quelconque des revendications précédentes, comportant un moyen d'identification, **caractérisé en ce qu'**il comprend des moyens empêchant une seconde utilisation du support adhésif (2) si celui-ci a déjà été utilisé pour le traitement d'une zone à traiter.

15. - Support adhésif (2) selon la revendication 14, **caractérisé en ce qu'**il comprend sur sa surface supérieure des marquages espacés d'une longueur égale à la largeur de la zone traitée par un tir individuel.

16. - Support adhésif (2) selon la revendication 14, **caractérisé en ce que** ledit support adhésif (2) est opaque.

17. - Support adhésif (2) selon la revendication 14, **caractérisé en ce qu'**il comprend un transpondeur.

18. - Support adhésif (2) selon la revendication 17, **caractérisé en ce que** le transpondeur fonctionne dans une bande de fréquence comprise entre 13 et 14 MHz

## Claims

1. System for dermatological treatment comprising an energy source (3) suitable for the biochemical activation of healing and at least one means of interaction (2) between the energy source and the patient, said means of interaction (2) being constituted of a continuous adhesive support that can be placed close to the area to be treated, **characterized in that** said support includes a means of identification (6) interacting without contact with a sensor (5) allowing the energy source to function only when the distance between the sensor and said means of identification is below a threshold value.

2. System for dermatological treatment according to claim 1, **characterized in that** the adhesive support (2) comprises at least 2 means of identification spaced apart at distance at least equal to a whole multiple of the width of the area treated by an individual shot.

3. System for dermatological treatment according to claim 2, **characterized in that** the means of identification are spaced at a distance equal to the width of the area treated by an individual shot.

4. System for dermatological treatment according anyone of the previous claims, **characterized in that** the means of identification interacts with the energy source through a contact.

5. System for dermatological treatment according anyone of the previous claims, **characterized in that** the parameters for the energy source to function are memorized in the means of identification and communicated to the device controlling of the energy source.

6. System for dermatological treatment according to anyone of the previous claims, **characterized in that** an information characterizes the state of the area to be treated, near which an adhesive support (2) including a means of identification is placed, the information being recorded in the means of identification, and relating to one of the following states:
- The area to be treated was not treated,
- The area to be treated is being treated,
- The treatment of the area to be treated has been completed.

7. System for dermatological treatment according to claim 6, **characterized in that** the control device receiving the information about the state of the area to be treated contains means of preventing a second use of the adhesive support including a means of identification when this has already been used once for treatment of an area to be treated.

8. System for dermatological treatment according to anyone of the previous claims, **characterized in that** it is associated to a wound closure device inserted in the edges of the wound, the absorption coefficient of which for the energy source being considered is less than that of the skin.

9. System for dermatological treatment according to anyone of the previous claims, **characterized in that** it contains a handpiece (1) incorporating a source of electromagnetic radiation for biochemical activation of healing and a means of interaction with said adhesive support (2).

10. System for dermatological treatment according to anyone of the previous claims, **characterized in that** it contains a handpiece (1) incorporating a laser source (3) and a means of interaction with said adhesive support (2).

11. System for dermatological treatment according to anyone of the previous claims, **characterized in that** it contains an energy source controlled by a microprocessor receiving a signal from a sensor able to interact with the means of identification of the dressing or the adhesive support (2).

12. System for dermatological treatment according to claim 9, **characterized in that** the handpiece (1) comprises a radiofrequency reader.

13. System for the dermatological treatment according to claim 12, **characterized in that** range of the radiofrequency antenna in the radiofrequency reader is less than 5 mm.

14. Adhesive support (2) for a system for dermatological treatment according to anyone of the previous claims, containing a means of identification, **characterized in that** it comprises means of preventing second use of the adhesive support (2) if the latter has already been used for the treatment of an area to be treated.

15. Adhesive support (2) according to claim 14, **characterized in that** it comprises on its upper surface markings spaced apart by a length equal to the width of the area treated by an individual shot.

16. Adhesive support (2) according to claim 14, **characterized in that** said adhesive support (2) is opaque.

17. Adhesive support (2) according to claim14, **characterized in that** it comprises a transponder.

18. Adhesive support (2) according to claim 17, **characterized in that** the transponder functions in a frequency band between 13 and 14 MHz.

## Patentansprüche

1. System zur dermatologischen Behandlung, das eine Energiequelle enthält (3), die zur biochemischen Aktivierung der Narbenbildung dient, sowie mindestens ein Interaktionsmittel (2) zwischen der Energiequelle und dem Patienten, wobei das Interaktionsmittel (2) aus einer kontinuierlichen, haftenden Vorrichtung besteht, die in die Nähe des zu behandelnden Bereichs bewegt werden kann, und das sich dadurch auszeichnet, dass diese Vorrichtung mit einem Identifikationsmittel (6) ausgestattet ist, das kontaktlos mit einem Sensor (5) interagiert, mit welchem die Energiequelle nur dann gesteuert wird, wenn der Abstand zwischen dem Sensor und dem oben genannten Identifikationsmittel einen bestimmten Schwellenwert unterschreitet.

2. System zur dermatologischen Behandlung gemäß dem Anspruch 1, das sich dadurch auszeichnet, dass die haftende Vorrichtung (2) mindestens 2 Identifikationsmittel enthält, die mindestens durch eine Länge voneinander getrennt sind, die einem Vielfachen der Breite des von einem Einzelschuss behandelten Hautbereichs entspricht.

3. System zur dermatologischen Behandlung gemäß dem Anspruch 2, welches sich dadurch auszeichnet, dass die Identifikationsmittel durch eine Länge voneinander getrennt sind, die der Breite des von einem Einzelschuss behandelten Hautbereichs entspricht.

4. System zur dermatologischen Behandlung gemäß obigen Ansprüchen, welches sich dadurch auszeichnet, dass das Interaktionsmittel über einen Kontakt mit der Energiequelle interagiert.

5. System zur dermatologischen Behandlung gemäß obigen Ansprüchen, das sich dadurch auszeichnet, dass die Funktionsparameter der Energiequelle im Identifikationsmittel gespeichert und der Vorrichtung zur Steuerung der Energiequelle übermittelt werden.

6. System zur dermatologischen Behandlung gemäß irgendeinem der obigen Ansprüche, das sich dadurch auszeichnet, dass eine Information den Zustand des zu behandelnden Hautbereichs beschreibt, in deren Nähe sich eine haftende Vorrichtung (2) mit einem Identifikationsmittel befindet, wobei die Information im Identifikationsmittel gespeichert wird und eine der folgenden Zustände der Haut beschreibt:
- Der zu behandelnde Hautbereich wurde noch nicht behandelt,
- Der zu behandelnde Hautbereich wird gerade behandelt,
- Die Behandlung des Hautbereichs ist nun abgeschlossen.

7. System zur dermatologischen Behandlung gemäß dem Anspruch 6, welches sich dadurch auszeichnet, dass die Steuerungsvorrichtung, das die Informationen bezüglich des zu behandelnden Hautbereichs empfängt, über die Fähigkeit verfügt, eine zweite Verwendung der mit einem Identifikationsmittel versehenen haftenden Vorrichtung zu verhindern, wenn diese bereits einmal zur Behandlung des zu behandelnden Hautbereichs verwendet worden ist.

8. System zur dermatologischen Behandlung gemäß irgendeinem der obigen Ansprüche, welches sich dadurch auszeichnet, dass es mit einer Vorrichtung zum Wundverschluss versehen ist, welche am Wundrand angebracht wird, wo der Absorptionsfaktor für die Energiequelle niedriger ist als auf der Haut.

9. System zur dermatologischen Behandlung gemäß irgendeiner der obigen Ansprüche, welches sich dadurch auszeichnet, dass es ein Handstück (1) enthält, in dem sich eine elektromagnetische Lichtquelle zur biochemischen Aktivierung der Wundheilung sowie ein Interaktionsmittel zur beschriebenen haftenden Vorrichtung (2) befinden.

10. System zur dermatologischen Behandlung gemäß irgendeinem der obigen Ansprüche, welches sich dadurch auszeichnet, dass es ein Handstück (1) enthält, in dem sich eine Laserquelle (3) und ein Interaktionsmittel mit der beschriebenen haftenden Vorrichtung befinden.

11. System zur dermatologischen Behandlung gemäß irgendeinem der obigen Ansprüche, welches sich dadurch auszeichnet, dass es eine Energiequelle enthält, welche von einem Rechner gesteuert wird, das ein Signal von einem Sensor empfängt, welcher mit dem Identifikationsmittel des Pflasters oder der haftenden Vorrichtung (2) interagieren kann.

12. System zur dermatologischen Behandlung gemäß dem Anspruch 9, welches sich dadurch auszeichnet, dass das Handstück (1) einen Radiofrequenzmesser enthält.

13. System zur dermatologischen Behandlung gemäß dem Anspruch 12, welches sich dadurch auszeichnet, dass die Reichweite der Antenne des Radiofrequenzmessers weniger als 5 mm beträgt.

14. Haftende Vorrichtung (2) eines Systems zur dermatologischen Behandlung gemäß irgendeinem der obigen Ansprüche, welches ein Identifikationsmittel enthält, das sich dadurch auszeichnet, dass es über die Fähigkeit verfügt, eine zweite Verwendung der haftenden Vorrichtung (2) zu verhindern, wenn diese bereits zur Behandlung eines zu behandelnden Hautbereichs eingesetzt worden ist.

15. Haftende Vorrichtung (2) gemäß dem Anspruch 14, welche sich dadurch auszeichnet, dass sie auf ihrer oberen Oberfläche Markierungen enthält, die in einem Abstand zueinander angeordnet sind, die der Länge des von einem Einzelschuss behandelten Hautbereichs entspricht.

16. Haftende Vorrichtung (2) gemäß den Ansprüchen 14, welche sich dadurch auszeichnet, dass diese Vorrichtung (2) undurchlässig ist.

17. Haftende Vorrichtung (2) gemäß den Ansprüchen 14, welche sich dadurch auszeichnet, dass sich in ihr ein Transponder befindet.

18. Haftende Vorrichtung (2) gemäß den Ansprüchen 17, welche sich dadurch auszeichnet, dass der Transponder in einem Frequenzbereich zwischen 13 und 14 MHz funktioniert.
